# EUROPEAN PATENT APPLICATION

(11) **EP 1 188 451 A1**
(43) Date of publication of application: **20.03.2002**
(21) Application number: 00307977.9
(22) Date of filing: 14.09.2000
(51) Int. Cl.: A61L 9/12, A61L 9/14

(54) **Dispensing liquids**

(71) Applicant: Slade, Brian, Ashford, Kent TN24 8BX (GB)
(72) Inventor: Slade, Brian, Ashford, Kent TN24 8BX (GB)
(74) Representative: Carter, Stephen John

(57) **Abstract**

A dispenser for a volatile material comprising a reservoir (8) for containing the material as a liquid, an emanating substrate (14) from which the volatile material can be released into the surrounding atmosphere, and a droplet dispenser (10) in fluid communication with the reservoir (8) for dispensing droplets of the volatile material from the reservoir onto the emanating substrate (14).

## Description

The present invention relates to devices for dispensing liquids, in particular, although not necessarily exclusively to devices for dispensing volatile materials in liquid form. It is envisaged that devices according to the invention will have particular application in dispensing liquid air fresheners.

The release of volatile liquids, eg. of air freshening materials and fragrances, into the atmosphere over a period of time can be achieved in a number of ways. Where a slow sustained dispersal of the material is required, it is known to expose a porous pad impregnated with the material or to provide a liquid reservoir from which a wick protrudes to draw up liquid from the reservoir, the liquid then evaporating from the exposed surface of the wick. These known dispersal methods have the advantage of low cost but they have a failing if the material to be dispersed is a mixture of substances with different volatilities, as is typical of fragrances. The components with greater volatility (e.g. "top-note" fragrances) then escape more readily than those with lower volatility (e.g. "bottom-" and "mid-note" fragrances) with the result that the character of the fragrance changes markedly over the period of use, which is clearly undesirable. If an impregnated porous pad is used, there is the further disadvantage that it cannot be readily seen when the volatile material is about to be exhausted.

In WO 00/30692 we describe an alternative form of dispenser for volatile liquids in which a downwardly extending dispersal screen formed by spaced threads is fed with a volatile liquid from a reservoir. The liquid is transported from the reservoir to the top of the screen via a capillary wick. This screen emanator has been found to provide a more uniform dispersal of fragrances comprised of components of different volatilities, because the nature of the screen limits the amount of liquid taken up from the reservoir at any one time so that both the top-notes and bottom-notes of the fragrance are quickly dispersed.

We now propose an alternative form of dispenser that can achieve a more regulated release of volatile material, particularly suited to dispensing highly volatile material in liquid form including fragrances having mainly top-note components.

Accordingly, in one aspect the invention provides a dispenser for a volatile material comprising a reservoir for containing the material as a liquid, an emanating substrate from which the volatile material can be released into the surrounding atmosphere, and a droplet dispenser in fluid communication with the reservoir for dispensing droplets of the volatile material from the reservoir onto the emanating substrate.

As with the screen emanator described in WO 00/30692, since only a small quantity of the volatile material is dispensed onto the substrate at any one time it is able to evaporate quickly. Moreover, the rate at which droplets are dispensed onto the substrate can be regulated to limit the rate at which the volatile material is released into the atmosphere. This avoids, for example, the very high, uncontrolled concentrations of top-note fragrances that would be experienced if using a conventional impregnated porous pad to dispense perfumes with a high top-note content.

The droplet dispenser may take the form of a passage of small cross-section, e.g. a micro-bore tube of small diameter, opening from the base of the reservoir. The diameter of the passage is selected as a function of the surface tension of the liquid in order that droplets are formed at the outer end of the passage and released onto the substrate, positioned below the passage outlet, one at a time. If the diameter of the passage is too great the liquid will flow constantly. If the diameter is too small no release of the liquid will occur. A suitable diameter for any particular liquid can be determined by experiment if necessary.

However, with this rather crude form of droplet dispenser it is not easy to regulate the rate of droplet formation, for example to vary the strength of the fragrance released into the atmosphere or to adapt the dispenser for different liquids. Moreover, the rate of droplet formation will also be dependent to some extent on the head of liquid in the reservoir.

In preferred forms, therefore, the droplet dispenser is arranged such that the hydrostatic pressure at the inner end (i.e. the end opening into the reservoir) of the outlet passage can be set independently of the head of liquid in the reservoir. Conveniently, this can be achieved with an arrangement in which the reservoir is closed at its top end, and includes an air supply tube through which air can enter the lower end of the reservoir. The base of the reservoir is also closed save for the outlet passage.

In a similar manner to a traditional "chicken-feeder" this form of reservoir finds an equilibrium position in which, due to a reduction in pressure in an air space formed above the liquid at its closed, upper end, the column of liquid in the chamber is supported by atmospheric pressure acting at the liquid/air interface present at the opening of the air supply tube to the lower end of the reservoir, as explained in more detail below.

The relative heights of the air/liquid interface at the opening of the air supply tube into the lower end of the reservoir and the inner end of the outlet passage will determine the pressure difference across the passage, and hence influence the rate of droplet formation. Thus, the height of the inner end of the passage is preferably adjustable relative to the air/liquid interface at the opening of the air supply tube into the reservoir. As the level of the inner end of the outlet passage is lowered relative to the liquid/air interface, causing the hydrostatic pressure at this inner end to be raised, the rate at which droplets are dispensed is increased. Conversely, the rate of droplet formation can be reduced, or even stopped, by raising the inner end of the outlet passage relative to the liquid/air interface.

In one preferred form of the reservoir, the air supply tube takes the form of an upright cylindrical tube extending within the reservoir, having an upper end open to the outside of the reservoir, its other, lower end opening into the reservoir. This construction can provide a particularly compact structure.

To allow the flow through the outlet passage to be more accurately controlled, it is preferably formed to act in the manner of siphon, the path of liquid through the passage first extending upwardly from its inner end before inverting and then extending downwardly to the outlet end of the passage. In this way, it is possible for the upper-most point in the liquid path through the passage to be raised above the level of the liquid/air interface to give a much slower rate of droplet formation.

To enhance the action of the siphon, a wick may be installed in the upwardly extending portion of the path through the outlet passage, such that the liquid is drawn up this portion by capillary action. The wick may also extend into the downwardly extending portion of the outlet passage, again creating a capillary flow through this portion. This results in a siphon arrangement that is not only self priming but which also, due to the resistance to flow presented by the wick, can serve to slow the flow of liquid through the outlet passage.

The droplet dispenser described above in the context of a dispenser for volatile liquids may also be used advantageously in other applications where a controllable release of a liquid from a reservoir is desired. Consequently, the present invention also provides, in a further aspect, such a droplet dispenser itself.

An embodiment of the invention is described below, by way of example, with reference to the accompanying drawings, in which:
Fig. 1 is a sectioned side view of a volatile material dispenser in accordance with an embodiment of the invention, showing a liquid reservoir part of the dispenser separated from a base part of the dispenser;
Fig. 2 is a sectioned side view of the dispenser of Fig. 1 with the reservoir part of the dispenser supported on the base; and
Fig. 3 is a further sectioned side view of the dispenser shown in an operating configuration.

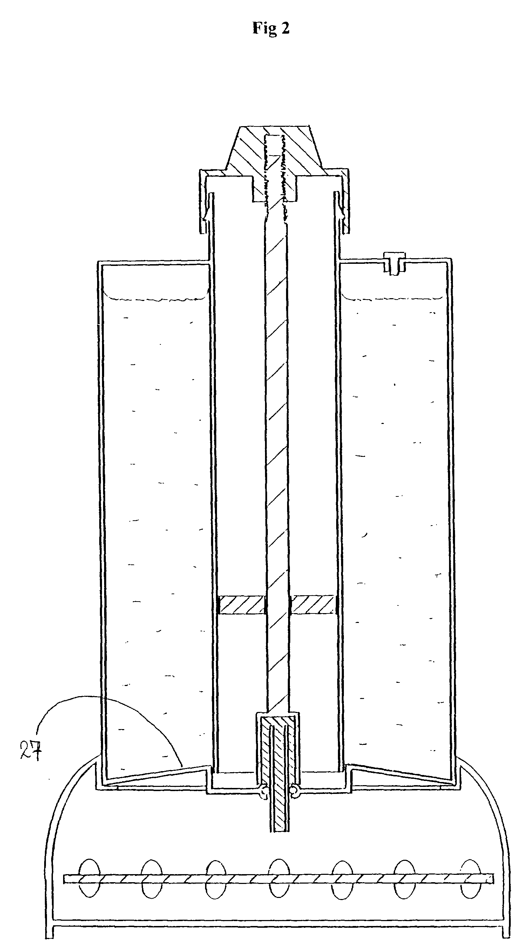

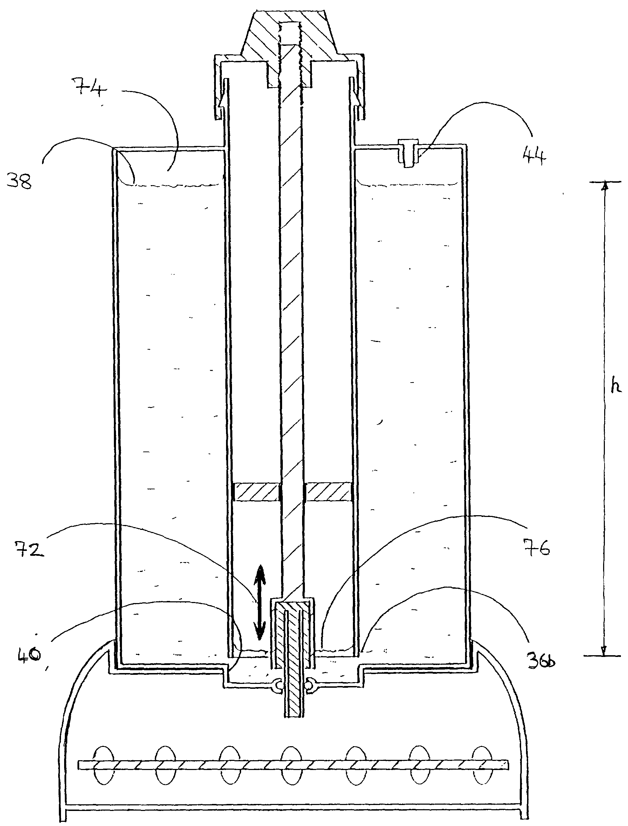

The illustrated dispenser is comprised of two main parts, a reservoir part 2 and a base part 4. The reservoir part is in the form of a cylindrical container 6 defining an annular reservoir 8 within. A droplet dispensing device 10, described in detail below, is integrated with the reservoir part 2. The base part 4 houses a substrate 14 onto which liquid from the reservoir 8 is dispensed by the droplet dispenser 10. The liquid, in the currently envisaged example a liquid fragrance, then emanates from the substrate into the surrounding atmosphere. In use, the reservoir part 2 is supported on the base part 4 as illustrated in Figs. 2 and 3.

Looking at the construction of the base part 4 in more detail, it comprises an enclosure 12 of generally cylindrical form. The substrate 14 is supported within the enclosure 12 above its base 16, so that both the top and bottom surfaces of the substrate 14 are exposed to the surrounding air. A series of apertures 18 around the circumference of the enclosure 12 provide a ready path for the fragrance to emanate from the enclosure 12 into the surrounding atmosphere.

The substrate 14 may take any of a number of suitable forms, but is preferably of a fibrous or porous material through which the liquid being dispensed rapidly spreads as it is released onto the substrate 14 from the droplet dispenser 10. Typically, therefore, the substrate will be of an open weave or other porous material having a large void ratio.

The substrate is also preferably of a thin web material no more than 1mm in thickness, more preferably about .25mm to .5mm in thickness, in order that the liquid is not easily trapped within the material.

The top face of the enclosure is formed with a generally circular recess 20 adapted to receive the lower end 22 of the reservoir part 2, the lower end of the reservoir part 2 fitting snugly against the cylindrical side wall 24 of the recess 20. A centre portion of the recesses face of the base part 4 is cut away to form an aperture 26 through which the droplet dispenser 10 can protrude into the interior of the enclosure 12.

As already noted above, the reservoir part 2 of the dispenser is of cylindrical form. More specifically, this part has a cylindrical outer side wall 30, closed at its opposite ends by top and bottom walls 32,34. A central, cylindrical air tube 36 extends concentrically within the outer wall 30, its upper end 36a protruding upwardly through the top wall 32 and its lower end 36b terminating adjacent the bottom wall 34.

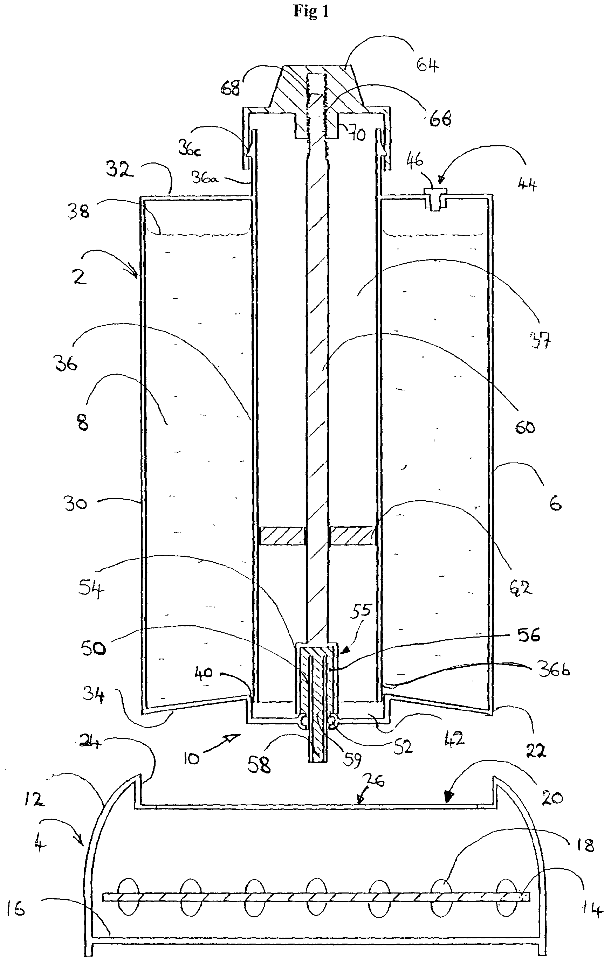

The generally annular reservoir 8 is defined between the outer wall 30, the air tube 36 and the top and bottom walls 32,34. In the configuration of the reservoir part 2 seen in Figs. 1 and 2, the bottom wall 34 is flexed inwardly so that the circumferential edge 40 of a recessed portion 42 in the centre of the bottom wall 34 engages the lower end 36b of the air tube 36, to form a liquid tight seal. This seal may be enhanced ,for example, by forming a mating groove and lip (not shown) respectively on the lower end 36b of the air tube 36 and the edge 40 of the recess 42 or vice versa. In this configuration, the reservoir 8 can be filled with liquid through an opening 44 in the top wall 32, which is subsequently closed by a bung 46.

In this example, at least the cylindrical outer wall 30 of the reservoir part is formed of a transparent material so that the level 38 of the liquid in the reservoir can be seen.

The droplet dispensing part 10 of the device will now be described. This part includes a micro-bore tube 50 that extends through the recessed portion 42 of the bottom wall 34. This tube is shown rather larger than its actual size in the figures for illustrative purposes, but in practice would typically have an internal diameter of 1-2mm (this has been found to give the desired droplet weight of about 0.08-0.15g currently envisaged). A seal 52 surrounds the tube 50 where it passes through the bottom wall 34.

The upper end of the micro-bore tube 50 is surrounded by an inverted cup-shape element 54 corresponding in cross-sectional shape to the micro-bore tube 50, but of slightly larger diameter so as to define a annular passage 56 between the outside surface of the micro-bore tube and the inside surface of the inverted cup 54. Thus, between them the micro-bore tube 50 and cup 54 define a siphon-like outlet passage from the reservoir, extending from the bottom edge of the cup 54 up through the annular passage 56 and then down through the micro-bore tube 50 to its outlet end 58. This outlet passage is packed with a wick 59 monofilament fibres to form a series of capillary passages extending along the length of the outlet passage. These fibre also serve to secure the micro-bore tube 50 in a fixed position within the cup 54, to form what might be referred to as a 'droplet dispensing siphon assembly' indicated generally by reference numeral 55.

The inverted cup element 54 is mounted on a vertically extending spindle 60 supported centrally within the air tube 36 by a support 62, mounted within the tube 36 and a control knob 64 mounted on the top of the tube 36. The support 62 in this example also serves as a seal to prevent any liquid escaping from the reservoir 8 through the air tube 36 should, for instance, the device be inverted. The control knob 64 is free to rotate on the top of the air tube 36, being retained by lip 36c, and, for reasons that will become apparent from the discussion of operation further below, freely allows air to enter the air tube 36 so that the pressure in its interior 37 remains substantially at atmospheric.

The upper end 66 of the spindle is externally threaded and engages a corresponding internal thread 68 of a boss 70 formed on the underside of the control knob 64. The support 62 grips the spindle 60 preventing it from rotating, so that when the control knob 64 is rotated in a first direction the threaded engagement causes the spindle 60, and hence the siphon assembly 55 to be raised relative to the air tube 36, as indicated by the arrow 72 in Figure 3. Rotation of the knob 64 in the opposite direction lowers the siphon assembly 55 relative to the air tube 36.

The operation of the device will now be described. As seen in Figs. 1 and 2, the bottom wall 34 of the reservoir part 2 is flexed upwardly to seal with the lower end 36b of the air tube. This serves to isolate the liquid in the reservoir 8 from the droplet dispenser 10.

In this 'sealed' configuration, the reservoir part 2 is first installed in the recess 20 in the top face of the base part 4 (see Fig. 2) and the seal is then broken by rotating the control knob 64 so as to push the spindle 60 and siphon assembly 55 downwards, the siphon part in turn pushing against the bottom wall 34 to move it away from the lower end 36b of the air tube. This allows the bottom wall 34 to spring into the position illustrated in Fig. 3 in which it is substantially flat and liquid to flows from the reservoir into the recess 42 in the bottom wall 34 and up into the lower end of the air tube 36.

In this state, there is a small, sealed air space 74 at the upper end of the reservoir 8 formed between the surface 38 of the liquid in the reservoir 8 and the top wall 32 and side wall 30. The air pressure in this space 74 is below atmospheric pressure. Specifically, in the equilibrium condition illustrated in Fig. 3, the pressure in this space is equal to atmospheric pressure less the hydrostatic pressure attributable to the head (h) of liquid above the lower end 36b of the air tube 36 where it opens into the reservoir 8. The air tube 36 is, as mentioned above, open to atmosphere at its upper end 36a, which of course means that the air in this tube is at atmospheric pressure. Consequently, atmospheric pressure acts on the surface 76 of the liquid at the air/liquid interface at the lower end of the air tube 36. Thus, a pressure balance is achieved between atmospheric pressure acting at this liquid/air interface 76 on the one hand, and the below atmospheric pressure in air space 74 combined with the hydrostatic pressure due to the head (h) of liquid above the liquid/air interface 38 on the other. In this equilibrium state, the relative levels of the two free liquid surfaces, namely the surface 38 at air space 74 and the surface 76 at the lower end of the air tube 36, are maintained.

Due to the capillary action exerted by the wick 59 within the outlet passage 56, liquid is drawn up through the annular passage 56 and then down through the micro-bore tube 50 to its outlet 58 where, due to the small diameter of the tube 50 and the surface tension of the liquid, a droplet forms. Once the droplet has developed it falls onto the centre of the underlying substrate 14, quickly spreads outwardly across the substrate 14, and subsequently emanates from the substrate 14 into the surrounding atmosphere.

The rate at which droplets are formed is a function of characteristics of the liquid, including surface tension and viscosity, characteristics of the siphon assembly 55, including the cross-sectional area of the passages through it and the capillary forces and resistance it creates, and also the pressure differential across the siphon assembly 55. Appropriate dimensions for the siphon assembly for any particular liquid can be determined by experiment if necessary, although the adjustment of the droplet rate described below means that one construction should be suitable for a wide variety of liquids.

As the liquid is gradually drawn off through the droplet dispensing siphon arrangement 55, the level of the liquid surface 38 at the top of the reservoir 8 drops. This results in an increase of the volume of the sealed air space 74 and a consequential drop in the air pressure in this space 74. This in turn creates an imbalance between the pressures acting on the two free liquid surfaces 38,76 within the reservoir 8. This imbalance causes air to flow into the reservoir 8 through the air tube 36, the air passing around the edge of the lower end 36b of the tube 36 to bubble upwardly through the liquid to the air space 74, to increase the pressure in that space until an equilibrium is once again restored. Once the equilibrium is restored, the hydrostatic pressure of the liquid at the level of the lower end of the air tube 36 is equal to atmospheric pressure once more.

Significantly, since there is atmospheric pressure acting at the liquid/air interface 76 at the lower end of the air tube 36, then in the equilibrium condition shown the hydrostatic pressure of the liquid at the level of this interface 76 is equal to atmospheric pressure. Thus, the hydrostatic pressure at this level in the reservoir 8, and hence the pressure differential across the droplet dispensing siphon assembly is maintained substantially constant irrespective of the level of the liquid in the reservoir, essentially eliminating this level as a factor in the rate of droplet formation.

The rate of droplet formation can, however, be influenced by adjusting the height of the droplet dispensing siphon assembly 55 relative to the air tube 36 and hence relative to the liquid/air interface 76 at the lower end of this tube 36. More specifically, as the siphon assembly 55 is raised relative to this liquid/air interface there is a resultant decrease in the pressure differential across the siphon assembly 55 and a corresponding decrease in the rate of droplet formation. On the other hand, if the siphon assembly 55 is lowered relative to the liquid/air interface 76, the pressure differential and hence droplet rate are increased. In this way, very good control of the rate at which e.g. a fragrance is released into the atmosphere can be achieved.

Since only a small amount of the liquid is released onto the substrate 14 at any one time, the described dispenser is particularly suited to dispensing liquids including highly volatile components, such as fragrances including top-note components. However, embodiments of the invention are not limited to dispensing such liquids and may be used in many other applications where a controlled release of a liquid in droplet form is required.

## Claims

1. A dispenser for a volatile material comprising:
a reservoir for containing the material as a liquid,
an emanating substrate from which the volatile material can be released into the surrounding atmosphere, and
a droplet dispenser in fluid communication with the reservoir for dispensing droplets of the volatile material from the reservoir onto the emanating substrate.

2. A dispenser according to claim 1, wherein the droplet dispenser comprises a passage of small cross-section opening from the base of the reservoir, the diameter of the passage being selected such that droplets are formed at the outer end of the passage and released onto the substrate one at a time.

3. A dispenser according to claim 2, wherein the droplet dispenser is arranged such that the hydrostatic pressure at the inner end the outlet passage can be set independently of the head of liquid in the reservoir.

4. A dispenser according to claim 3, wherein the reservoir is closed at its top end, and includes an air supply tube through which air can enter the lower end of the reservoir.

5. A dispenser according to any one of the preceding claims, wherein the rate at which droplets are dispensed is adjustable.

6. A dispenser according to claim 5 when dependent on claim 4, wherein the height of the inner end of the outlet passage is adjustable relative to the air supply tube.

7. A dispenser according to claim 4 or 6, wherein the air supply tube takes the form of an upright cylindrical tube extending within the reservoir, having an upper end open to the outside of the reservoir, its other, lower end opening into the reservoir.

8. A dispenser according to any one of claims 2 to 4 , 6 or 7, wherein said outlet passage is formed to act in the manner of siphon, the path of liquid through the passage first extending upwardly from its inner end before inverting and then extending downwardly to the outlet end of the passage.

9. A dispenser according to claim 8, wherein a wick is disposed in the upwardly extending portion of the path through the outlet passage, such that the liquid is drawn up this portion by capillary action.

10. A dispenser according to claim 9, wherein the wick extends into the downwardly extending portion of the outlet passage.
